# EUROPEAN PATENT APPLICATION

(11) **EP 4 227 661 A2**
(43) Date of publication of application: **16.08.2023**
(21) Application number: 23382033.1
(22) Date of filing: 17.01.2023
(51) Int. Cl.: G01N 1/40, B01L 7/00, C12Q 1/6806, G01N 33/18, G01N 35/10

(54) **SYSTEM FOR THE ANALYSIS OF WASTEWATER OR ENVIRONMENTAL WATER FOR THE DETECTION OF VIRUSES, BACTERIA OR EUKARYOTIC MICROORGANISMS**

(30) Priority: 11.02.2022 ES 202200055 U
(71) Applicant: Empresa Municipal de Aguas de Gijón, 33212 Gijón (ES); Magna Dea S.L, 33002 Oviedo (ES); Universidad De Oviedo, 33003 Oviedo (ES); Consorcio de Aguas de Asturias, 33007 Oviedo (ES)
(72) Inventor: VILLA MIGUEL, ALBERTO, OVIEDO (ES); PEREZ ALVAREZ, JULIO ANTONIO, OVIEDO (ES); FERNANDEZ NUEVO, JORGE, GIJÓN (ES); CASO SANCHEZ, JOSE ANGEL, GIJÓN (ES); MENENDEZ RODRIGUEZ, PEDRO, GIJÓN (ES)
(74) Representative: Pons

(57) **Abstract**

The present invention relates to a system comprising sample extraction and concentration equipment (3), intended to extract an amount of water through a sanitation manifold and concentrate said amount until a sample is obtained to carry out the qRT-PCR test, and the results of which are sent to a thermocycling equipment (7) which receives the processed water samples in automatic pipetting pieces of equipment (5, 6) and improves the processing of said water samples by carrying out cycles at different temperatures, passing finally to a control system that analyses them and issues alerts when the extracted data shows a risk of outbreak of any virus, bacterium or eukaryotic microorganism that can be located by detecting its nucleic acid or remotely and in real time.

## Description

### TECHNICAL SECTOR

The present invention integrates the fields of robotic engineering, molecular biology and microbiology for the extraction, concentration and preparation of wastewater or environmental samples, to which a qRT-PCR test is performed and the results of which are transferred to a control system that analyses same and issues alerts when the extracted data shows a risk of an outbreak of any virus, bacteria or eukaryotic microorganism that can be located by detecting its nucleic acid or remotely and in real time.

### BACKGROUND OF THE INVENTION

Currently, the devices that exist on the market for the concentration, preparation and performance of qRT-PCR tests on wastewater or environmental samples are installed in laboratories and their operation is independent of each other.

To date, a manual extraction of the sample must be carried out in the sanitation network manifolds, a process that must be carried out by qualified personnel. These samples are transferred to the laboratories where the appropriate processes to carry out the qRT-PCR tests are applied for the detection of the virus, bacteria or eukaryotic microorganism of interest. Normally the extraction points for these samples may be far from the places where the rest of the process is carried out, having to adopt measures in these cases to preserve the conditions of the sample until its handling in the destination laboratory. Although freezing is accepted, much more reliable results are usually obtained with samples that are analysed while still fresh, immediately after extraction.

In this sense, there is no specifically designed equipment or assembly on the market that combines all the instruments required to carry out the complete process autonomously, automatically, continuously, remotely and with real-time alarm issuance. The present model comes to cover the automation deficiencies present in the market, also speeding up early detection and alarm issuance in case of reaching values related to outbreaks of the disease of interest where appropriate.

### DESCRIPTION OF THE INVENTION

The equipment claimed is made up of a sample extraction and concentration equipment, two automatic pipetting pieces of equipment, a thermocycler, a computer and a robotic arm. All of them are arranged on a bench of approximately two square metres in area and with a lower shelf to store the required consumables. This set of pieces of equipment works together perfectly coordinated from the intended IT equipment or computer.

Working separately, the pieces of equipment that make up the claimed model have specific functions to carry out different actions independently. These are:
- Sample extraction and concentration equipment: this device extracts an amount of water from the sanitation manifold or environmental water and concentrates it until obtaining a suitable sample for carrying out the qRT-PCR test.
- Automatic pipetting pieces of equipment: In these pieces of equipment, the samples collected from wastewater or environmental water are prepared, mixing them with different reagents until obtaining the amount of nucleic acid required to later carry out the preparation of the mixtures of the qRT-PCR reactions.
- Thermocycler: equipment where cycles are carried out at different temperatures for the amplification of various areas of the target DNA using the qRT-PCR technique, or for other types of reactions based on these nucleic acids, such as sequencing.
- Robotic arm to move the tubes and plates with samples between the different pieces of equipment.
- Computer: containing the programs for coordinating the operation of all the components of the system, as well as that for alarm issuance in the qRT-PCR tests, in the event of obtaining various established values.
- Workbench: Wherein all the equipment that makes up the system, as well as the consumables, will be located.

With this utility model, it is therefore possible to automate the entire process of early detection of possible outbreaks associated with viruses, bacteria or eukaryotic microorganisms, which are traceable by their genetic material (DNA or RNA), from the extraction of the wastewater or environmental water sample, until obtaining the result of the qRT-PCR test, its visualisation and continuous control in real time and remotely. This model can perform various screening tests using qRT-PCR without the need for personnel intervention.

It is a set of pieces of equipment which, given the optimisation of its functions, is arranged on a suitable surface to facilitate its transfer and placement at different sampling points. In this way, on a bench of approximately two square metres of surface area, all the pieces of equipment that make up the set are arranged, being able to be located without problems in most of the existing points that can be used for monitoring in a wastewater treatment network or the different locations for environmental waters of interest (rivers, lakes, reservoirs, etc.).

### BRIEF DESCRIPTION OF THE DRAWINGS

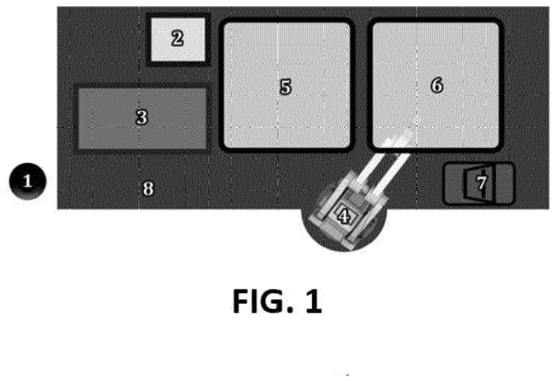

Figure 1 shows a plan view of the arrangement of the equipment on the equipment's workbench, according to the present invention, being:
1. Opening to the manifold of the sanitation network or to the environmental water.
2. Computer.
3. Sample extraction and concentration equipment.
4. Robotic arm.
5. Automatic pipetting equipment 1 for the extraction of the nucleic acid of interest (DNA or RNA).
6. Automatic pipetting equipment 2 for the preparation of qRT-PCR reactions.
7. Thermocycling equipment.
8. Bench.

### PREFERRED EMBODIMENT OF THE INVENTION.

The present invention, represented schematically in Figure 1, has assembled on a bench (8) having two heights, with a surface area of approximately two square metres for each one, a sample extraction and concentration equipment (3), from the opening to the sanitation manifold or to the environmental water to be analysed (1), made on the floor of the sampling point facility.

Next, and in parallel, the two automatic pipetting pieces of equipment (5, 6) will be placed. And centred on the long side of the table, in front of them, there is a robotic arm (4) that will move the sample tubes and plates between the different pieces of equipment. Next to these pieces of equipment is the thermocycling equipment (7) to which the final sample arrives with the mixture of nucleic acid and corresponding reagents for carrying out the qRT-PCR test.

The coordination of the system, as well as the analysis of the results and alarm issuance is carried out through the computer (2) placed on the bench together with the rest of the devices.

## Claims

1. A system for analysing wastewater or environmental water from a sanitation manifold for the detection of viruses, bacteria or eukaryotic microorganisms, **characterised in that** it comprises:
- a workbench (8),
- an opening (1) for a sanitation manifold,
- a sample extraction and concentration equipment (3) supported on the workbench (8) and which is designed to extract a quantity of water through the opening (1) of the sanitation manifold and concentrate said quantity until obtaining a sample to perform the qRT-PCR test,
- a number of automatic pipetting pieces of equipment (5, 6) supported on the workbench (8) and which receive the water samples from the sample extraction and concentration equipment (3) to process same, and
- a thermocycling equipment (7) which receives the processed water samples from the automatic pipetting pieces of equipment (5, 6) and improves the processing of said water samples by carrying out cycles at different temperatures.

2. The system for analysing wastewater or environmental water according to claim 1, further comprising:
- a robotic arm (4) responsible for automatically transferring the samples in tubes or water sample plates between the sample extraction and concentration equipment (3), the automatic pipetting pieces of equipment (5, 6) and the thermocycling equipment (7), and
- a computer (2) electronically connected to the robotic arm (4), to the sample extraction and concentration equipment (3), to the automatic pipetting pieces of equipment (5, 6) and to the thermocycling equipment (7), configured to govern the electronic operation of said elements and to issue alarms when the samples reach established concentration and processing values.

3. The system for analysing wastewater or environmental water according to claim 1, wherein the automatic pipetting pieces of equipment (5, 6) extract nucleic acid of interest from the water sample and prepare qRT-PCR reactions with said water samples respectively.

4. The system for analysing wastewater according to claim 1, wherein the workbench (8) has an area of two square metres and includes a lower level for the collection of consumables.
